(19) Europäisches Patentamt
European Patent Office
Office européen des brevets

(11) **EP 3 831 801 B1**

(12) **EUROPEAN PATENT SPECIFICATION**

(45) Date of publication and mention
of the grant of the patent:
**31.01.2024 Bulletin 2024/05**

(21) Application number: **20211491.4**

(22) Date of filing: **03.12.2020**

(51) International Patent Classification (IPC):
**C07C 45/37** [(2006.01)]    **C07C 37/54** [(2006.01)]
**C07C 45/39** [(2006.01)]    **D21C 5/00** [(2006.01)]

(52) Cooperative Patent Classification (CPC):
(C-Sets available)
**D21C 5/00; C07C 37/54; C07C 45/37; C07C 45/39;**
Y02E 50/10; Y02P 20/584        (Cont.)

(54) **A METHOD FOR CO-PRODUCING MONOPHENOLS AND CELLULOSE BY CATALYTIC OXIDATION OF BIOMASS OVER A TRANSITION METAL OXIDE**

VERFAHREN ZUR CO-HERSTELLUNG VON MONOPHENOLEN UND CELLULOSE DURCH KATALYTISCHE OXIDATION VON BIOMASSE ÜBER EIN ÜBERGANGSMETALLOXID

PROCÉDÉ DE COPRODUCTION DE MONOPHÉNOLS ET DE CELLULOSE PAR OXYDATION CATALYTIQUE DE LA BIOMASSE SUR UN OXYDE DE MÉTAL DE TRANSITION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.12.2019 CN 201911220057**

(43) Date of publication of application:
**09.06.2021 Bulletin 2021/23**

(73) Proprietor: **Guangzhou Institute of Energy Conversion,**
**Chinese Academy of Sciences**
**Guangzhou 510640 (CN)**

(72) Inventors:
• **WANG, Chenguang**
  **Guangzhou, 510640 (CN)**
• **ZHU, Yuting**
  **Guangzhou, 510640 (CN)**
• **MA, Longlong**
  **Guangzhou, 510640 (CN)**
• **LIU, Jing**
  **Guangzhou, 510640 (CN)**
• **LV, Wei**
  **Guangzhou, 510640 (CN)**
• **ZHANG, Qi**
  **Guangzhou, 510640 (CN)**

(74) Representative: **KIPA AB**
**Drottninggatan 11**
**252 21 Helsingborg (SE)**

(56) References cited:
**CN-A- 108 164 407    JP-A- 2015 089 884**

• **TRAN THI HA ET AL: "Copper Oxide Nanomaterials Prepared by Solution Methods, Some Properties, and Potential Applications: A Brief Review", INTERNATIONAL SCHOLARLY RESEARCH NOTICES, vol. 2014, 17 December 2014 (2014-12-17), pages 1-14, XP055794559, DOI: 10.1155/2014/856592 Retrieved from the Internet: URL:http://downloads.hindawi.com/archive/2014/856592.xml>**

(52) Cooperative Patent Classification (CPC): (Cont.)

C-Sets
**C07C 37/54, C07C 39/02;**
**C07C 45/37, C07C 47/565;**
**C07C 45/37, C07C 47/575;**
**C07C 45/37, C07C 47/58;**
**C07C 45/39, C07C 47/565;**
**C07C 45/39, C07C 47/575;**
**C07C 45/39, C07C 47/58**

C-Sets
**C07C 37/54, C07C 39/02;**
**C07C 45/37, C07C 47/565;**

**Description**

**Technical Field**

The present invention relates to the technical field of biomass-derived chemicals, in particular to a method for the co-production of monophenol compounds and cellulose by transition metal oxide catalytic oxidation of biomass.

**Background**

Research on the substitution of lignocellulosic biomass for fossil resources to prepare chemicals and materials has attracted wide attention. Lignin, as one of the three main components of lignocellulosic biomass and rich in aromatic ring structures, is an ideal source for the preparation of monophenolic chemicals of high commercial value. However, due to its complex structure, it is difficult to be utilized efficiently. At present, main technologies for lignin conversion are pyrolysis, hydrogenation and oxidation, among which oxidation has the advantages of mild reaction conditions and high selectivity of monophenols.

The main products of lignin oxidation are aromatic aldehydes, i.e. vanillin, syringaldehyde and p-hydroxy benzaldehyde. These aromatic aldehydes have important applications in the fields of food, cosmetics, medicine, pesticide and chemical industry. Therefore, lignin oxidation technology has been widely studied. For instance, Praveen et al. used niobium oxalate as a catalyst to catalyze alkali lignin to obtain aromatic aldehyde in yield of 8.9% (vanillin, 6.6%; syringaldehyde, 2.3%) (L. Das, P. Kolar, J. A. Osborne, R. R. Sharma-Shivappa, J. J. Classen, Selective Oxidation of Lignin into Aromatic Aldehydes Using Niobium Oxalate. Transactions of the ASABE, 2016, 59, 727-735). Li et al. found porphyrin (Co(TPPS$_4$)) efficient in catalyze different biomass (including corn straw after enzymatic hydrolysis by steam explosion, lignocellulose residue rich in lignin after ethanol production) with the highest yield of aromatic aldehydes of 12.8% (Y. Li, J. Chang, Y. Ouyang. Selective Production of Aromatic Aldehydes from Lignin by Metalloporphyrins/H2O2 System. Advanced Materials Research, 2013, 805-806, 273-276). The substrates used in the above studies were extracted lignin or biomass pretreated under harsh conditions. The yield of monophenols was generally low because the lignin structure was heavily damaged during extraction or pretreatment, during which more stable condensation structure that is difficult to be depolymerized were formed. Moreover, the oxidant used in the above works was hydrogen peroxide which is not conducive to industrial production for its too strong oxidizing property.

The yield of monophenols can be significantly increased when raw biomass is used as raw material. For example, monophenols yield of 29.4% (vanillin selectivity, 71.8%) was obtained by oxidation of pine powder with oxygen as the oxidant in NaOH solution (Y. T. Zhu, J. Liu, Y. H. Liao, W. Lv, L. L. Ma, C. G. Wang. Degradation of Vanillin During Lignin Valorization Under Alkaline Oxidation. Topics in Current Chemistry, 2018, 376:29). However, the yield of monophenols obtained by the current oxidation process is lower than the theoretical yield, and the yield of cellulose is relatively low. The use of catalyst is expected for further improvement the yield of monophenols. Transition metal oxides such as CuO are commonly used catalysts for catalytic oxidation reactions. However, so far, the co-production of monophenols and high-purity cellulose from lignocellulosic biomass by catalytic oxidation of transition metal oxides is rarely reported.

CN108164407 discloses a method for preparing monophenols, small-molecule organic acids and cellulose by aqueous oxidation of lignocellulose biomass, using CuSO$_4$·5H$_2$O as catalyst. In JP2015089884 there is disclosed a method for producing a lignin monomer, which comprises irradiating by microwave radiation a mixture of a lignin component-containing plant material and a metal compound to decompose the lignin component-containing plant material. Tran et al (Int Schol Res Notice, 2014, 1-14) discloses copper oxide nanomaterials prepared by applying microwaves to copper salts in an alkaline medium to generate CuO by sequential transformation.

In summary, using lignocellulosic biomass as raw material to prepare monophenolic chemicals and co-produce high-purity cellulose is an effective measure to deal with the current problems of shortage of fossil resources and environmental damage. However, existing oxidation technologies have problems such as unsafe oxidant, low product concentration and low yield of monophenols.

**Summary**

The present invention, defined by the appended claims, aims to overcome the drawbacks of existing technology, and to provide a green, safe, mild-condition, and simple method of transition metal oxide catalytic oxidation of biomass to phenolic compounds, which uses lignocellulose as raw material, oxygen as oxidant, transition metal oxides as catalyst; and co-produces monophenols and cellulose in moderate conditions, thereby the lignocellulose can be efficiently utilized.

A method for co-production of monophenol compounds and cellulose by transition metal oxide catalytic oxidation of biomass is provided comprising:

(a) a reactor is loaded with pretreated dry biomass, transition metal oxide and alkali metal hydroxide solution and then sealed, the reactor being evacuated and refilled with $O_2$, the $O_2$ pressure being kept at 0.1~3 MPa, and the mixture is allowed to react at 80~200 °C for 1~180 min under stirring, and then cooled down at room temperature to obtain a suspension, wherein the dry biomass is pretreated by treatment with anhydrous ethanol and refluxing for 8 h, then dried at 50~60 °C, and the content of transition metal oxide is 1-100% of the weight of the dry biomass;

(b) the suspension obtained in step (a) is centrifuged to separate clear supernatant from solid residue, the supernatant being acidified to pH 2-3, organic solvent being added to the acidified solution to extract phenolic compounds and the organic phase being gathered; and the residual acid and water in the organic solution are removed; and monophenols can be obtained by vacuum distillation of the organic phase; and

(c) the solid residue obtained in step (b) is placed in a sieve of 800 meshes, and the catalyst is rinsed out with water while the biomass residue retains on the sieve, the aqueous solution containing catalyst is filtered or centrifuged to recycle the spent catalyst, the biomass residue is washed with acid aqueous solution to remove the residual catalyst until the solution is clear, and continuously washed with water until the pH value of waste liquid reaches 7 and then the biomass residue is dried.

At step (b), strong base-weak acid salt is added to neutralize inorganic acid remaining in the organic solution, and the organic solution is dried by anhydrate $Na_2SO_4$. The organic solvent used in step (b) is preferably dichloromethane, chloroform, tetrahydrofuran, ethyl acetate or acetone. The obtained monophenols are mainly vanillin, syringaldehyde, p-hydroxy benzaldehyde.

The dry biomass is preferably softwood, hardwood, grass, or agro-forestry residue, and more preferably pine, eucalyptus, Chinese pennisetum, bagasse, or corn stalk. The lignin content of pine is about 23%, correspondingly, the theoretical maximum yield of monophenols of pine is 38%. The lignin content and theoretical maximum yield of monophenols of eucalyptus are about 26% and 50%, respectively.

Before the oxidation reaction, dry biomass is suffered to Soxhlet extraction to remove the ethanol-soluble oil and pigment. The typical steps of the soxhlet extraction are as follows: biomass is treated with anhydrous ethanol and refluxing for 8 h, then dried at 50~60 °C.

The hydroxide of the alkali metal is preferably NaOH, KOH, or $Mg(OH)_2$. The weight percent concentration of the aqueous solution of the hydroxide of the alkali metal is preferably at the range of 2.5-15%.

Preferably, the solid-liquid ratio of dry biomass to aqueous solution of hydroxide of alkali metal is at the range of 1:20~50.

Preferred transition metal oxides include CuO, $TiO_2$, NiO, $Co_2O_3$, $Fe_2O_3$, $Fe_3O_4$ and ZnO.

More preferably, the transition metal oxide is CuO nanoparticles with diameter of 40 to 200 nm, and the content of CuO nanoparticles is preferably 20-40% of the weight of the dry biomass.

Preferred reaction conditions in step (a) are listed as follows: reaction temperature: 120~180 °C, agitation speed: 50~1000 rpm, reaction time: 10~120 min, oxygen pressure: 0.5-1.5 MPa.

The present invention is advantageous and beneficial especially in that:

the raw materials used in the invention is of wide source, low cost and easy to harvest;

the catalysts used in the present invention are commercially available, which can be reused and easy to be industrialized; and

under mild conditions, lignin can be efficiently transformed into monophenols with aromatic aldehyde as the main component and co-producing high-purity cellulose, the yield of monophenols up to 46% and the selectivity of aromatic aldehyde up to 90%. Moreover, the yield and purity of cellulose are up to 90% and 98%, respectively.

## Detailed Description of the Invention

The present invention is described in detail below.

The equipment and materials mentioned in the present invention are commercially available, unless otherwise stated. Unless otherwise specified, the percent sign "%" referred to in the present invention refers to the weight percentage.

The yield and selectivity of monophenol, yield and purity of cellulose are calculated by the following formula.

$$\text{Yield of monophenol} = (\text{weight of monophenol} / \text{weight of lignin in pine})$$
$$\times 100\%$$

$$\text{Selectivity of aromatic aldehyde} = (\text{weight of aromatic aldehyde} / \text{weight of all monophenols}) \times 100\%$$

$$\text{Yield of cellulose} = (\text{weight of cellulose retained} / \text{weight of cellulose in pine}) \times 100\%$$

$$\text{Selectivity of cellulose} = (\text{weight of cellulose retained} / \text{weight of biomass residue}) \times 100\%$$

**Example 1**

A method for co-production of monophenol compounds and cellulose by transition metal oxide catalytic oxidation of biomass is provided in this example. The method has the following steps.

(a) In a 50 mL autoclave reactor, 0.5 g of pretreated dry pine powder, 0.1 g (the weight of 20% of biomass) of 40 nm CuO, 25 mL of NaOH aqueous solution of 7.5 %, were put into the reaction vessel and then sealed. The reactor was evacuated and refilled with $O_2$, the $O_2$ pressure was kept at 1 MPa. The reaction mixture was heated at 160 °C for 60 min under stirring speed of 400 rpm. After cooled down at room temperature, the reactor was open and a suspension was obtained.

(b) The suspension obtained in step (a) was centrifuged to separate clear supernatant and solid residue. The supernatant was acidified with hydrochloric acid (36% aqueous solution) until pH value was 2~3. Dichloromethane was added to the acidified solution to extract phenolic compounds. The dichloromethane phase was gathered. $NaHCO_3$ was added into dichloromethane solution to neutralize residue hydrochloric acid, and then $Na_2SO_4$ was added to remove the remaining water. Finally, monophenols was obtained by vacuum distillation of the dichloromethane phase.

(c) The solid residue obtained in step (b) was placed in a sieve of 800 meshes, and the catalyst was rinsed out with water while the biomass residue retained on the sieve. The aqueous solution containing catalyst was filtered or centrifuged to obtain the spent catalyst, and the spent catalyst can be used in the next batch. The biomass residue was washed with hydrochloric acid aqueous solution to remove the residual catalyst until the solution clear, continue washed with water until the pH value of waste liquid reached 7. Finally, the biomass residue was dried.

The yield, selectivity and purity of products are listed in Table 1.

**Examples 2 to 5**

The preparation process is substantially the same as Example 1, except that the catalyst 40 nm CuO was replaced with 50 nm $TiO_2$, 20 nm $Fe_2O_3$, 50 nm ZnO and 20 nm $Al_2O_3$. The yield, selectivity and purity of products are listed in Table 1.

Table 1. Results of catalytic oxidation of pine over different metal oxides.

| Examples | Catalyst | Yield of monophenols (%) | Selectivity of vanillin (%) | Yield of cellulose (%) | Purity of cellulose (%) |
|---|---|---|---|---|---|
| Example 1 | 40nm CuO | 36 | 73 | 80 | 95 |
| Example 2 | 50nm $TiO_2$ | 22 | 70 | 48 | 95 |
| Example 3 | 20nm $Fe_2O_3$ | 17 | 72 | 30 | 92 |
| Example 4 | 50nm ZnO | 18 | 71 | 35 | 98 |

(continued)

| Examples | Catalyst | Yield of monophenols (%) | Selectivity of vanillin (%) | Yield of cellulose (%) | Purity of cellulose (%) |
|---|---|---|---|---|---|
| Example 5 | 20nm $Al_2O_3$ | 21 | 70 | 50 | 96 |

It can be seen in Table 1 that all the selected transition metal oxides can catalyze the oxidation of biomass to monophenols and retain a portion of cellulose. Compared with other metal oxides, the nano CuO exhibits superior performance in catalytic oxidation of pine to produce monophenols and co-production of high-purity cellulose.

**Examples 6 to 8**

The preparation process is substantially the same as Example 1, except that the catalyst 40 nm CuO was replaced with 200 nm CuO, 10 $\mu$m CuO and 75 $\mu$m CuO. The yield, selectivity and purity of products are listed in Table 2.

Table 2. Results of catalytic oxidation of pine over CuO with different particle sizes.

| Examples | Catalyst | Yield of monophenols (%) | Selectivity of vanillin (%) | Yield of cellulose (%) | Purity of cellulose (%) |
|---|---|---|---|---|---|
| Example 1 | 40 nm | 36 | 73 | 80 | 95 |
| Example 6 | 200 nm | 32 | 75 | 75 | 95 |
| Example 7 | 10 $\mu$m | 26 | 72 | 62 | 95 |
| Example 8 | 75 $\mu$m | 25 | 70 | 60 | 94 |

It can be seen in Table 2 that the yield of monophenols and cellulose increases gradually as the particle size of CuO particles decreases, and the 40nm CuO shows the best catalytic activity.

**Examples 9 to 12**

The preparation process is substantially the same as Example 1, except that the dosage of 40 nm CuO was changed to 10%, 40%, 60% and 80% of the dosage of biomass. The yield, selectivity and purity of the products are shown in Table 3.

Table 3. Results of catalytic oxidation of pine over 40 nm CuO with different dosage.

| Examples | Dosage 40 nm CuO (%) | Yield of monophenols (%) | Selectivity of vanillin (%) | Yield of cellulose (%) | Purity of cellulose (%) |
|---|---|---|---|---|---|
| Example 1 | 20 | 36 | 73 | 80 | 95 |
| Example 9 | 10 | 31 | 76 | 65 | 98 |
| Example 10 | 40 | 25 | 73 | 68 | 97 |
| Example 11 | 60 | 22 | 74 | 68 | 92 |
| Example 12 | 80 | 18 | 75 | 65 | 93 |

As can be seen in Table 3, the yields of monophenols and cellulose increases and then decreases with the increase of the dosage of 40 nm CuO, and the best catalytic performance is achieved at the dosage of 20%.

**Examples 13 to 14**

The preparation process is substantially the same as Example 1, except that the catalyst 40 nm CuO was replaced by catalyst reused at the second time and third time. The yield, selectivity and purity of the products are shown in Table 4.

Table 4. Results of catalytic oxidation of pine over 40 nm CuO.

| Example | 40 nm CuO used time | Yield of monophenols (%) | Selectivity of vanillin (%) | Yield of cellulose (%) | Purity of cellulose (%) |
|---|---|---|---|---|---|
| Example 1 | First time | 36 | 73 | 80 | 95 |
| Example 13 | Second time | 30 | 76 | 78 | 98 |
| Example 14 | Third time | 29 | 77 | 76 | 97 |

As can be seen in Table 4, nano CuO shows no obvious reduction of catalytic activity after three recycles, indicating its high reusability.

**Examples 15 to 19**

The preparation process is substantially the same as Example 1, except that the biomass pine was replaced by eucalyptus, Chinese pennisetum, bagasse, corn stalk, furfural residue of corn stalk. The yield, selectivity and purity of the products are shown in Table 5.

Table 5. Results of catalytic oxidation of different biomass over 40 nm CuO.

| Examples | Biomass | Yield of monophenols (%) | Selectivity of vanillin (%) | Selectivity of syringaldehyde (%) | Selectivity of p-hydroxy benzaldehyde (%) | Yield of cellulose (%) | Purity of cellulose (%) |
|---|---|---|---|---|---|---|---|
| Example 1 | pine | 36 | 73 | 0 | 0 | 80 | 95 |
| Example 15 | eucalyptus | 46 | 24 | 66 | 0 | 80 | 98 |
| Example 16 | Chinese pennise | 25 | 40 | 25 | 11 | 90 | 97 |
| Example 17 | bagasse | 20 | 37 | 25 | 15 | 40 | 96 |
| Example 18 | corn stalk | 15 | 50 | 20 | 15 | 40 | 95 |
| Example 19 | furfural residue | 10 | 30 | 20 | 12 | 10 | 92 |

As can be seen in Table 5, nano CuO is efficient in catalyze oxidation of various biomasses to monophenols with aromatic aldehydes as main product and co-producing high-purity cellulose. Among the biomasses, the highest yield of monophnol achieves when eucalyptus is used as raw material, while Chinese pennisetum gives the highest yield of cellulose.

**Example 20**

The preparation process is substantially the same as Example 1, except that step (a) was modified as follows: In a 50 mL autoclave reactor, 0.5 g of pretreated dry pine powder, 0.2g (the weight of 40% of biomass) of 40 nm CuO, 25 mL of NaOH aqueous solution of 2.5 %, were put into the reaction vessel and then sealed. The reactor was evacuated and refilled with $O_2$, the $O_2$ pressure was kept at 0.5 MPa. The reaction mixture was heated at 120 °C for 120 min under stirring speed of 50 rpm. After cooled down at room temperature, the reactor was open and a suspension was obtained. The yield of monophenols was 15%, selectivity of vanillin was 72%; and the yield and purity of cellulose was 85% and 81%, respectively.

**Example 21**

The preparation process is substantially the same as the example 1, except that step (a) was modified as follows: In a 50 mL autoclave reactor, 0.5 g of pretreated dry pine powder, 0.1 g (the weight of 20% of biomass) of 40 nm CuO, 10 mL of NaOH aqueous solution of 15 %, were put into the reaction vessel and then sealed. The reactor was evacuated and refilled with $O_2$, the $O_2$ pressure was kept at 1.5 MPa. The reaction mixture was heated at 180 °C for 10 min under stirring speed of 1000 rpm. After cooled down at room temperature, the reactor was open and a suspension was obtained. The yield of monophenols was 25%, selectivity of vanillin was 70%; and the yield and purity of cellulose was 60% and 74%, respectively.

**Claims**

1. A method for co-production of monophenols and cellulose by transition metal oxide catalytic oxidation of biomass, comprising:

      (a) loading a reactor with pretreated dry biomass, transition metal oxide and alkali metal hydroxide solution and then the reactor is sealed, evacuated and refilled with O2, the O2 pressure being kept at 0.1~3 MPa, and the mixture is allowed to react at 80~200 °C for 1~180 min under stirring, and then cooled down at room temperature to obtain a suspension, wherein the dry biomass is pretreated by treatment with anhydrous ethanol and refluxing for 8 h, then dried at 50~60 °C, and the content of transition metal oxide is 1~100% of the weight of the dry biomass;
      (b) the suspension obtained in step (a) is centrifuged to separate clear supernatant from solid residue, the supernatant being acidified to pH 2-3, an organic solvent being added to the acidified solution to extract phenolic compounds and the organic phase being gathered; the residual acid and water in the organic solution are removed and monophenols are obtained by vacuum distillation of the organic phase; and
      (c) the solid residue obtained in step (b) is placed in a sieve, and the catalyst is rinsed out with water while the biomass residue is retained on the sieve, the aqueous solution containing catalyst is filtered or centrifuged to recycle the spent catalyst, the biomass residue is washed with acid aqueous solution to remove the residual catalyst until the solution is clear, and continuedly washed with water until the pH value of waste liquid reaches 7 and then the biomass residue is dried.

2. The method of claim 1, wherein the dry biomass is lignocellulosic biomass, and wherein the lignocellulosic biomass is selected from a group consisting of hardwood, softwood, grass and agro-forestry residue.

3. The method of claim 1, wherein the alkali metal hydroxide is selected from a group consisting of NaOH, KOH and $Mg(OH)_2$.

4. The method of claim 1, wherein the solid-liquid ratio of dry biomass to aqueous solution of hydroxide of alkali metal is 1:20~50.

5. The method of claim 1, wherein the transition metal oxide is selected from a group consisting of CuO, TiO2, NiO, Co2O3, Fe2O3, Fe3O4 and ZnO.

6. The method of claim 5, wherein the transition metal oxide is CuO nanoparticles.

7. The method of claim 6, wherein the particle size of CuO nanoparticles is 40~200 nm.

8. The method of claim 6, wherein the content of CuO nanoparticles is 20-40% of the weight of the dry biomass.

9. The method of claim 1, wherein the reaction temperature is 120~180 °C.

10. The method of claim 1, wherein the stirring speed is 50~1000rpm.

**11.** The method of claim 1, wherein the reaction time is 10~120 min.

**12.** The method of claim 3, wherein the concentration of alkali metal hydroxide aqueous solution is 2.5~15 wt%.

**Patentansprüche**

**1.** Ein Verfahren zur gleichzeitigen Herstellung von Monophenolen und Cellulose durch katalytische Oxidation von Biomasse mit:

(a) Beladen eines Reaktors mit vorbehandelter trockener Biomasse, einem Übergangsmetalloxid und einer Alkalimetallhydroxidlösung, anschließender Versiegelung, Entleerung und Wiederbefüllung des Reaktors mit $O_2$, wobei der $O_2$-Druck auf 0,1~3 MPa gehalten wird und das Gemisch bei 80~200 °C für 1~180 min unter Rühren reagieren kann und anschließend auf Raumtemperatur abgekühlt wird, um eine Suspension zu erhalten, wobei die trockene Biomasse über eine Behandlung mit wasserfreiem Ethanol vorbehandelt wird und für 8 h zurückfließt, dann bei 50~60 °C getrocknet wird und einen Übergangsmetalloxid-Gehalt von 1-100 % des Trockenbiomassegewichts aufweist,

(b) Zentrifugieren der in Schritt (a) gewonnenen Suspension zur Trennung des klaren Überstands vom festen Rückstand, wobei der Überstand auf pH 2~3 angesäuert, ein organisches Lösemittel der angesäuerten Lösung zur Extraktion phenolischer Bestandteile zugesetzt und die organische Phase aufgefangen wird, die restliche Säure und das Wasser in der organischen Lösung entfernt und die Monophenole durch Vakuumdestillation der organischen Phase gewonnen werden, und

(c) Platzieren des in Schritt (b) gewonnenen Feststoffs in einem Sieb, Ausspülen des Katalysators mit Wasser, während der Biomassenrückstand im Filter verbleibt, Filtern der wässrigen Lösung mit dem Katalysator oder Zentrifugieren zum Recyceln des eingesetzten Katalysators, Auswaschen des Biomassenrückstands mit saurer wässriger Lösung zur Entfernung von Katalysatorrückständen bis die Lösung klar ist, kontinuierliches Waschen mit Wasser, bis der pH-Wert der verbleibenden Flüssigkeit 7 erreicht und anschließendes Trocknen des Biomassenrückstands.

**2.** Das Verfahren gemäß Anspruch 1, wobei es sich bei der trockenen Biomasse um lignocellulosehaltige Biomasse handelt und die lignocellulosehaltige Biomasse zwischen Hartholz, Weichholz, Gras und agroforstwirtschaftlichen Rückständen gewählt wird.

**3.** Das Verfahren gemäß Anspruch 1, wobei das Alkalimetallhydroxid zwischen NaOH, KOH und $Mg(OH)_2$ gewählt wird.

**4.** Das Verfahren gemäß Anspruch 1, wobei das Verhältnis von trockener Biomasse zu wässriger Lösung des Alkalimetallhydroxids bei 1:20~50 liegt.

**5.** Das Verfahren gemäß Anspruch 1, wobei das Übergangsmetalloxid zwischen CuO, $TiO_2$, NiO, $Co_2O_3$, $Fe_2O_3$, $Fe_3O_4$ und ZnO gewählt wird.

**6.** Das Verfahren gemäß Anspruch 5, wobei es sich bei dem Übergangsmetalloxid um CuO-Nanopartikel handelt.

**7.** Das Verfahren gemäß Anspruch 6, wobei die Größe der CuO-Nanopartikel bei 40~200 nm liegt.

**8.** Das Verfahren gemäß Anspruch 6, wobei der Gehalt an CuO-Nanopartikel 20~40 % des Gewichts der trockenen Biomasse entspricht.

**9.** Das Verfahren gemäß Anspruch 1, wobei die Reaktionstemperatur bei 120~180 °C liegt.

**10.** Das Verfahren gemäß Anspruch 1, wobei die Rührgeschwindigkeit bei 50~1000 U/min liegt.

**11.** Das Verfahren gemäß Anspruch 1, wobei die Reaktionszeit 10~20 min beträgt.

**12.** Das Verfahren gemäß Anspruch 3, wobei die Konzentration der wässrigen Lösung des Alkalimetallhydroxids bei 2,5~15 Gew. % liegt.

**Revendications**

1. Procédé de coproduction de monophénols et de cellulose par oxydation catalytique de biomasse sur un oxyde de métal de transition, comprenant les étapes suivantes :

(a) un réacteur est chargé avec de la biomasse sèche prétraitée, un oxyde de métal de transition et une solution d'hydroxyde de métal alcalin, puis le réacteur est fermé, mis sous vide et rerempli avec de l'$O_2$, la pression d'$O_2$ étant maintenue entre 0,1 et 3 MPa, et le mélange est laissé à réagir à une température comprise entre 80 et 200 °C pendant de 1 à 180 min sous agitation, puis est refroidi à température ambiante pour obtenir une suspension, où la biomasse sèche est prétraitée par traitement avec de l'éthanol anhydre et reflux pendant 8 h, puis séchée à une température comprise entre 50 et 60 °C, et la teneur en oxyde de métal de transition est comprise entre 1 et 100 % de la masse de la biomasse sèche ;

(b) la suspension obtenue à l'étape (a) est centrifugée pour séparer un surnageant limpide d'un résidu solide, le surnageant étant acidifié à un pH compris entre 2 et 3, un solvant organique étant ajouté à la solution acidifiée pour extraire les composés phénoliques et la phase organique étant recueillie ; l'acide résiduel et l'eau résiduelle dans la phase organique sont éliminés et des monophénols sont obtenus par distillation sous vide de la phase organique ; et

(c) le résidu solide obtenu à l'étape (b) est placé sur un tamis, et le catalyseur est extrait par rinçage à l'eau tandis que le résidu de biomasse est retenu sur la tamis, la solution aqueuse contenant le catalyseur est filtrée ou centrifugée pour recycler le catalyseur consommé, le résidu de biomasse est lavé avec une solution aqueuse acide pour éliminer le catalyseur résiduel jusqu'à ce que la solution soit limpide, et ensuite lavé avec de l'eau jusqu'à ce que la valeur de pH du liquide résiduaire atteigne 7, puis le résidu de biomasse est séché.

2. Procédé selon la revendication 1, dans lequel la biomasse sèche est de la biomasse lignocellulosique, et où la biomasse lignocellulosique est sélectionnée parmi un groupe constitué de bois dur, de bois tendre, de graminées et de résidu d'agrosylviculture.

3. Procédé selon la revendication 1, dans lequel l'hydroxyde de métal alcalin est sélectionné parmi un groupe constitué de NaOH, de KOH et de Mg(OH)$_2$.

4. Procédé selon la revendication 1, dans lequel le rapport solide-liquide de biomasse sèche sur solution aqueuse d'hydroxyde de métal alcalin est comprise entre 1:20 et 1:50.

5. Procédé selon la revendication 1, dans lequel l'oxyde de métal de transition est sélectionné parmi un groupe constitué de CuO, de TiO$_2$, de NiO, de Co$_2$O$_3$, de Fe$_2$O$_3$, de Fe$_3$O$_4$ et de ZnO.

6. Procédé selon la revendication 5, dans lequel l'oxyde de métal de transition est des nanoparticules de CuO.

7. Procédé selon la revendication 6, dans lequel la taille de particules des nanoparticules de CuO est comprise entre 40 et 200 nm.

8. Procédé selon la revendication 6, dans lequel la teneur en nanoparticules de CuO est comprise entre 20 et 40 % de la masse de la biomasse sèche.

9. Procédé selon la revendication 1, dans lequel la température de réaction est comprise entre 120 et 180 °C.

10. Procédé selon la revendication 1, dans lequel la vitesse d'agitation est comprise entre 50 et 1 000 tr/min.

11. Procédé selon la revendication 1, dans lequel le temps de réaction est compris entre 10 et 120 min.

12. Procédé selon la revendication 3, dans lequel la concentration de la solution aqueuse d'hydroxyde de métal alcalin est comprise entre 2,5 et 15 % en poids.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 108164407

- JP 2015089884 B

**Non-patent literature cited in the description**

- **L. DAS ; P. KOLAR ; J. A. OSBORNE ; R. R. SHARMA-SHIVAPPA ; J. J. CLASSEN.** Selective Oxidation of Lignin into Aromatic Aldehydes Using Niobium Oxalate. *Transactions of the ASABE,* 2016, vol. 59, 727-735
- **Y. LI ; J. CHANG ; Y. OUYANG.** Selective Production of Aromatic Aldehydes from Lignin by Metalloporphyrins/H2O2 System. *Advanced Materials Research,* 2013, 805-806, 273-276

- **Y. T. ZHU ; J. LIU ; Y. H. LIAO ; W. LV ; L. L. MA ; C. G. WANG.** Degradation of Vanillin During Lignin Valorization Under Alkaline Oxidation. *Topics in Current Chemistry,* 2018, vol. 376, 29
- **TRAN et al.** *Int Schol Res Notice,* 2014, vol. 1-14